Europäisches Patentamt

European Patent Office

Office européen des brevets

(11) Publication number: **0 594 879 A1**

## EUROPEAN PATENT APPLICATION

(21) Application number: **92118369.5**

(22) Date of filing: **28.10.92**

(51) Int. Cl.5: **C12P 21/08**, C12N 5/20, G01N 33/577, G01N 33/68, //C12N15/57

(43) Date of publication of application:
**04.05.94 Bulletin 94/18**

(84) Designated Contracting States:
**BE CH DE FR GB IT LI NL SE**

(71) Applicant: **IDEMITSU KOSAN COMPANY LIMITED**
**1-1, Marunouchi 3-chome**
**Chiyoda-ku**
**Tokyo 100(JP)**

(72) Inventor: **Kawabata, Takahiro, c/o Idemitsu Kosan Co., Ltd.**
**1280, Kamiizumi,**
**Sodegaura-shi**
**Chiba(JP)**
Inventor: **Miyayama, Tetsuo, c/o Idemitsu Kosan Co., Ltd.**
**1-1, Marunouchi 3-chome**

**Chiyoda-ku, Tokyo(JP)**
Inventor: **Yokoi, Ryoji**
**Sofare KII 205,**
**2245-2, Goi**
**Ichihara-shi, Chiba(JP)**
Inventor: **Irie, Shinji, c/o Idemitsu Kosan Co., Ltd.**
**1280, Kamiizumi,**
**Sodegaura-shi**
**Chiba(JP)**
Inventor: **Nakayama, Kouji, c/o Idemitsu Kosan Co., Ltd.**
**1280, Kamiizumi,**
**Sodegaura-shi**
**Chiba(JP)**

(74) Representative: **Türk, Gille, Hrabal, Leifert**
**Brucknerstrasse 20**
**D-40593 Düsseldorf (DE)**

(54) **Cathepsin L-specific monoclonal antibodies, hybridomas for producing the same and a method of using the same.**

(57) A monoclonal antibody is disclosed which is reactive specifically with human procathepsin L or with any of the following peptides corresponding to parts of the primary amino acid sequence of human procathepsin L:

a peptide comprising the 94th to 107th amino acid sequence: Gly-Phe-Gln-Asn-Arg-Lys-Pro-Arg-Lys-Gly-Lys-Val-Phe-Gln;

a peptide comprising the 112th to 125th amino acid sequence: Tyr-Glu-Ala-Pro-Arg-Ser-Val-Asp-Trp-Arg-Glu-Lys-Gly-Tyr;

a peptide comprising the 282nd to 295th amino acid sequence: Gly-Tyr-Gly-Phe-Glu-Ser-Thr-Glu-Ser-Asp-Asn-Asn-Lys-Tyr;

a peptide comprising the 297th to 310th amino acid sequence: Leu-Val-Lys-Asn-Ser-Trp-Gly-Glu-Glu-Trp-Gly-Met-Gly-Gly; and

a peptide comprising the 308th to 321st amino acid sequence: Met-Gly-Gly-Tyr-Val-Lys-Met-Ala-Lys-Asp-Arg-Arg-Asn-His.

Also disclosed are a hybridoma characterized by producing the monoclonal antibody and a method of detecting human cathepsin L characterized by employing the monoclonal antibody to detect cathepsin L in a specimen from a human.

It is possible to specifically detect human procathepsin L by utilizing a monoclonal antibody according to the present invention. Accordingly, the antibody may be utilized for the diagnosis and therapy of diseases related with human procathepsin L.

EP 0 594 879 A1

# FIG. 1

Primer of 5' end
Primer of 3' end
cDNA of Procathepsin L
(Prepared by PCR)

pUC118
Hind III
Hinc II
Bam HI
EcoR I
Amp^r
ori

Cleavage with Hinc II

Linkage

Hind III
cDNA of Procathepsin L

pUC118–procat L
Amp^r
ori
BamH I
EcoR I

## FIELD OF THE INVENTION

The present invention relates to human procathepsin L-specific monoclonal antibodies, hybridomas for producing the same and a method of using the same.

These monoclonal antibodies are useful for the field in the detecting,the diagnosis and the therapy.

## BACKGROUND OF THE INVENTION

Lysosomes contain a wide variety of proteases and are connected with the degradation of foreign proteins. Cathepsin L belongs to the group of cysteine proteases contained in lysosomes.

Cathepsin B1 in the serum, which is now separated into cathepsin B and cathepsin L, has been reported to be correlated with the progress of cervical cancer, particularly in the early stages thereof (Gynecologic oncology, Vol.7, pp.1-17,1979). Also, evidence suggests an increase in the activity of cathepsin L in gastric tissue of cancer patients (OKAYAMA Medical Journal, 100,89-95,1988) and the importance of cathepsin L in osteoporosis (63rd Annual Meeting of the Japanese Biochemical Society, Tokushima University, Enzyme Lab.) For the foregoing reasons, it has been desired to establish a method of specifically detecting human (pro) cathepsin L in humans.

To date, the base sequence of cDNA for human cathepsin L has been reported by Gottesman, etc. (Biochem. J., Vol.253, pp.303-306, 1988), but no polyclonal antibody nor monoclonal antibody thereto has been acquired yet.

Therefore, cathepsin L is determined by its activity of decomposing certain substrates. In addition, no specific substrate is known for procathepsin L, and thus no method has been proposed for either direct determination (analysis) or quantitative determination of procathepsin L (pro and mature segments).

## SUMMARY OF THE INVENTION

First, the present invention is intended to provide monoclonal antibodies which are reactive specifically with human cathepsin L and peptides corresponding to some parts of the primary amino acid sequence of human procathepsin L, including those comprising the 94th to 107th amino acid sequence:
Gly-Phe-Gln-Asn-Arg-Lys-Pro-Arg-Lys-Gly-Lys-Val-Phe-Gln,
the 112th to 125th amino acid sequence:
Tyr-Glu-Ala-Pro-Arg-Ser-Val-Asp-Trp-Arg-Glu-Lys-Gly-Tyr,
the 282nd to 295th amino acid sequence:
Gly-Tyr-Gly-Phe-Glu-Ser-Thr-Glu-Ser-Asp-Asn-Asn-Lys-Tyr,
the 297th to 310th amino acid sequence:
Leu-Val-Lys-Asn-Ser-Trp-Gly-Glu-Glu-Trp-Gly-Met-Gly-Gly and
the 308th to 321st amino acid sequence:
Met-Gly-Gly-Tyr-Val-Lys-Met-Ala-Lys-Asp-Arg-Arg-Asn-His.

Second, it is intended to provide hybridomas characterized by producing said monoclonal antibodies.

Third, it is intended to provide a method of detecting human cathepsin L in specimens from humans.

## BRIEF DESCRIPTION OF THE DRAWINGS

Fig. 1 diagrams the construction of pUC118 containing cDNA for human cathepsin L.

Fig. 2 diagrams the construction of the expression vectors pGEX-3X and pKK233-2 which contain cDNA for human cathepsin L.

Fig. 3 illustrates the reactivity between procathepsin L in human serum and the monoclonal antibodies in Western blotting.

## DETAILED DESCRIPTION OF THE INVENTION

The monoclonal antibodies according to the present invention may be prepared by, for example, the following procedures:

1) Preparation of antigens

(1) Preparation and expression of cDNA for human procathepsin L

The cDNA for human available for use according to the present invention may be any one commercially available as cDNA libraries from CLONTECH Lab., Inc. which derives from a human kidney, liver, placenta, etc. Alternatively it may be obtained via preparation of mRNA in a conventional manner and then use of the corresponding reverse transcriptase. The oligonucleotide (primer) comprising the object cDNA for human procathepsin L may be synthesized in a conventional manner, and the gene amplification (by PCR) may be conducted under general conditions (according to the directions for the particular kit used). For example, the primer may be mixed with the cDNA prepared from an organ such as a kidney, and the mixture is then subjected 25 to 60 times to a reaction which has a cycle consisting of heating at 950°C for 30 sec. to 1 min., at 37-55°C for 30 sec. to 2 min. and at 70-74 °C for 1 to 3 min.

Then follows a successive process which comprises agarose electrophoresis, preparation of the band of cDNA for human procathepsin L through cutting-out, transformation into E.coli, treatment with restriction enzymes, joining reactions, blunting and so on. According to the manner described in Molecular Cloning, second edition (Maniatis, T. et al., published by Cold Spring Harbor Laboratory,1989) or the direction for the particular kit, the expression vector may be pGEX-3X, pKK233-2 (both commercially available from Pharmacia Co., Ltd.) or the like, and the general-purpose plasmid may be pUC118 (commercially available from Takara-Shuzo Co., Ltd.)

(2) Purification of GST-human procathepsin L protein

The host E. coli may be any one, and preferably is Y1091 strain, JM105 strain, JM109 strain or the like. The cultivation is appropriately conducted at a temperature of 23-42 °C, preferably 36-38°C. An appropriate inducing time with isopropyl D-thiogalactopyranoside (IPTG) ranges from 1 to 18 hours, preferably from 1 to 4 hours. Cell crushing may be done in a conventional manner, and particularly preferred is the process using an ultrasonic crusher or a French press. Further, the solubilizing agent is preferably urea, guanidine hydrochloride, SDS or the like, while the anion exchange resin for purification desirably is DEAE or QAE type or the like. Any carrier for gel filtration may be used. No particular limitation is set on the type of the ultrafilter and ultrafiltration membrane used.

(3) Preparation of synthetic peptides of human procathepsin L

The object amino acid sequence of from 10 to 20 amino acid residues which construct the pro or mature segment of human procathepsin L is synthesized on the basis of the base sequence of cDNA for human procathepsin L (Biochem. J., Vol. 253, pp. 303 -306, 1988), and is joined to a carrier protein. Here, the carrier protein may be, for example, KLH, BSA, OVA, PLL or the like. The joining of the peptide and the carrier protein may be done by GAD, ECDI or MBS process or the like.

2) Preparation of monoclonal antibodies

(1) Immunity

First, mammals, especially primates, rodents (mice, rats, rabbits, etc.), cattle, sheep, goats, dogs and so on are rendered immune to GST-procathepsin L produced by E. coli using genetic recombination procedures, or to peptide-carrier combinations constructed by joining a partially synthesized peptide on the basis of the entire primary amino acid sequence of procathepsin L to a carrier protein such as bovine serum albumin (BSA) or KLH. Mice were used as the mammals in the hereunder presented examples according to the present invention.

(2) Cell fusion

The immunized animal cells, for example, spleen cells from a mouse, are fused with myeloma cells in a conventional manner. Here, the myeloma cells may be any derived from mice, including NS-0, NS-1, P3U1, etc, and SP2/0-Ag14 strain was actually used in the hereunder presented examples according to the present invention.

4

(3) Screening

The screening for the hybridomas formed by the cell fusion mentioned above may be carried out by the enzyme antibody technique using an alkaline phosphatase, glucose oxidase, peroxidase, $\beta$-D-galactosidase or the like as the secondary antibody, and O-phenylenediamine, 3,3-diaminobenzidine, 2,2-azinobis-O-dianisidine, 4-chloronaphthol,4-aminoantipyrine or the like as the substrate.

(4) Cloning

The cloning may be made according to any of the hitherto known methods including limiting dilution-culture method, soft agar culture method, etc.

(5) Production of monoclonal antibodies

In order to produce monoclonal antibodies using the purified hybridomas, for example, one may carry out a method for the mass culture of a hybridoma in which a serum or serum-free medium is used, or one in which a hydridoma is inoculated into the abdomen of a mouse and the abdominal water is recovered.

(6) Purification of the monoclonal antibodies

The purification of the monoclonal antibodies may be performed according to any of the hitherto known procedures utilizing protein A-Sepharose affinity column, protein G-Sepharose affinity column, high performance liquid chromatography and so on.

By the use of the monoclonal antibodies to human procathepsin L according to the present invention, cathepsin L may be detected by any of the publicly known immunological detecting methods (sandwich ELISA, etc.)

An explanation will be made hereunder of an embodiment of the detection procedures.

A first monoclonal antibody to human cathepsin L according to the present invention (first antibody) is immobilized on an appropriate insoluble support (for example, a plastic container); hereunder this is referred to as "immobilized antibody." Next, the surface of the insoluble support is coated with an appropriate material (for example, bovine serum albumin) in order to prevent non-specific adsorption from occurring between the insoluble support and the reagent or specimen to be determined.

The thus prepared insoluble support on which the first antibody is immobilized is co-incubated with a specimen suspected of containing cathepsin L which has been pre-diluted to an appropriate concentration.

Generally the dilution of the specimen is made with PBS, whereas it may be conducted with an acetate buffer solution of pH 4.0-6.0 (preferably pH 5.0) in the case where the specimen is human serum, thereby making it possible to improve its reactivity with the antibody by incubation after a neutralization treatment.

The immobilized antibody and the human cathepsin L in the specimen bind to each other during this incubation.

Then, after being washed with an appropriate wash, a solution of the other monoclonal antibody labeled with an appropriate labeling material, for example, an enzyme (hereunder referred to as "second antibody"), is contacted with the human cathepsin L binding to the solid phase antibody on the insoluble support. This contact is maintained for a certain period of time and at a certain temperature for reaction with the second antibody.

The mixture is washed with an appropriate wash, and then the quantity is determined of the labeling material which was used to label the second antibody binding to the solid phase antibody on the insoluble support, via the intervening human cathepsin L.

Thus, the human cathepsin L in the specimen may be detected based on said quantity.

Here, the insoluble support available for use may be, for example, polystyrene, polyethylene, polypropylene, polyester, crosslinked dextran, polysaccharide or other high molecular material as well as paper, glass, metal, agarose and a mixture thereof. Also it may be of any various shape, including tray-like, spherical, etc.

In addition, enzymes, fluorescent materials, luminous materials, radioactive materials and so on may be used for their advantages as a labeling material for the antibody to be labeled.

The enzymes available for use include peroxidase, alkaline phosphatase and $\beta$-D-galactosidase. Embodiments of the fluorescent materials are fluorescein isothiocyanate and so on. Isolucinol, lucigenin, etc. may be employed as the luminous materials, while the radioactive materials include $^{125}I$, $^{131}I$, $^{14}C$, $^{3}H$ and so on. Of course, the enzymes, fluorescent materials, luminous materials, and radioactive materials are not limited to those exemplified above; any other one under those categories may be used so far as it may be employed in a method for immunological determination.

If peroxidase is employed as the labeling enzyme, then the substrate may be $H_2O_2$, and O-phenylenediamine, 5-aminosalicylic acid or the like may be chosen as the color reagent. On the other hand, O-nitrophenyl phosphate or the like may be used as the substrate when the labeling enzyme is an alkaline phosphatase, and 4-methylumbelliferyl-$\beta$-D-galactopyranoside or the like may be used as the substrate when the labeling enzyme is $\beta$-D-galactosidase.

The monoclonal antibodies according to the present invention may be employed for the diagnosis of cancer and other diseases through detection of human cathepsin L, wherein the antibody is reacted with a specimen from a human which contains human cathepsin L including human procathepsin L or the like; for example, one from a patient suffering from a cancer connected with human cathepsin L, such as, gastric cancer, bronchogenic cancer, pancreatic cancer, hepatic cancer, cervical cancer, ovarian tumour or the like, or from a disease connected with aging such as osteoporosis.

The specimens from humans include human serum, urine, cells, etc.

The present invention will be explained in more detail hereunder with reference to preparations and examples.

Preparations 1 and 2

1) Preparation of cDNA for human procathepsin L from human cDNA library

A DNA was prepared from a human kidney cDNA library (manufactured by CLONETECH Lab., Inc.) in a conventional manner (reference should be made to, for example, "Molecular Cloning" supra.), and, on the basis of the base sequence of cDNA for human procathepsin L (Biochem. J., Vol.253, pp.303-306,1988) (reference should be made to the LIST Of SEQUENCES given later), there were synthesized the oligonucleotides (21 mer) at each of the 5' and 3' ends of cDNA for human procathepsin L; see the following:

5' end: 5' TGCCTTTTGCCTGGGAATTGC 3' (21 mer)
3' end: 5' AAGATGAATTCCTCCCATGCA 3' (21 mer)

Next, the DNA was amplified by subjecting the above DNA from the cDNA library and the synthetic nucleotides to the PCR method, which was done 50 times with a cycle composed of 1 minute at 95°C, 2 minutes at 55°C and 3 minutes at 72°C using a kit available from Takara Shuzo Co., Ltd. and according to the directions attached thereto. The resulting reaction product was subjected to 0.6% agarose electrophoresis, and about 1 kb of DNA was prepared by cutting out of the gel in a conventional manner.

2) Expression of cDNA for human procathepsin L

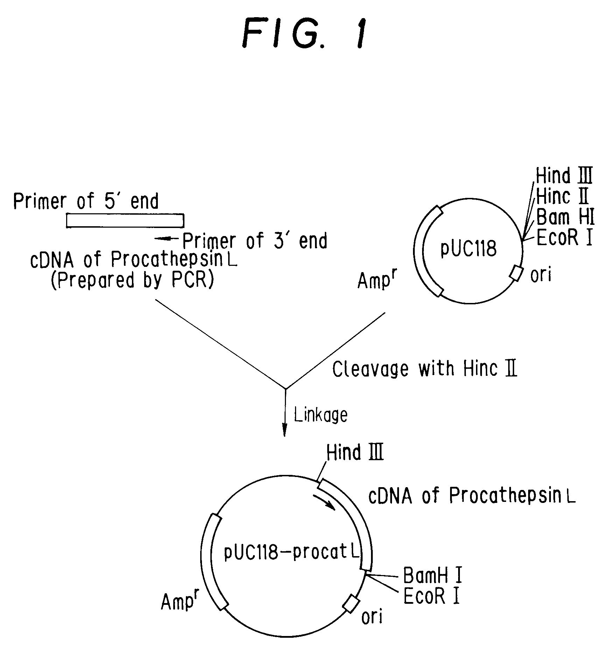

Making use of a ligation kit of Takara Shuzo Co., Ltd, the DNA from the above 1) was linked with plasmid pUC118 which had been cleaved with restriction enzyme HincII, and thereafter E. coli JM109 was transformed with the plasmid to get E. coli comprising the plasmid pUC118 which contains cDNA for human procathepsin L; see Fig.1.

E. coli cannot yield the recombinant human procathepsin L which all the N-terminal amino acids are involved (JBC., Vol.264, 20487-20495,1980), so it is a necessity to yield one lacking the 12 N-terminal amino acids of its pre or pro segment. Therefore the DNA from the above 1) was cleaved with restriction enzyme AvaII, and the protruding ends were made blunt with DNA polymerase (using a blunting kit manufactured by Takara Shuzo Co., Ltd.) followed by linking with phosphated BamHI linker (8 mer).

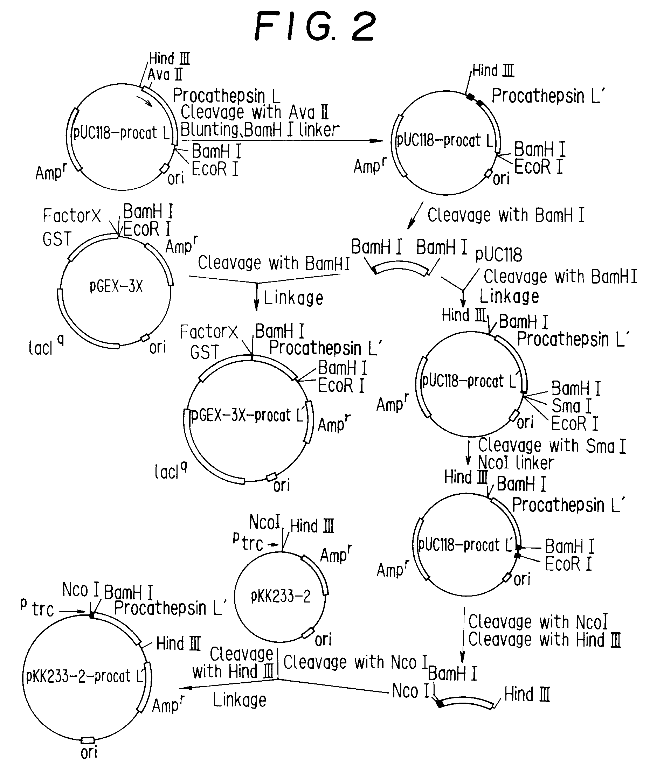

The cDNA for human procathepsin L was harvested as a BamHI fragment which was then bound to the BamHI site on glutathione S-transferase (GST) fused type expression vector pGEX-3X, and E. coli JM109 and Y1091 were thereafter harvested which yield GST-human procathepsin L fusion protein (Fig. 2).

In the same manner, the BamHI fragment was linked with plasmid pUC118 into which NcoI linker (8 mer) was introduced at the SmaI cleavage site, and the NcoI-HindIII fragment was linked with expression vector pKK233-2 to get E. coli JM109 which yields human procathepsin L protein only (Fig. 2). Here, the expression vector is not necessarily limited to pGEX-3X or pKK233-2 mentioned above. Further, other types of E. coli may be used as the host in addition to the above-mentioned ones.

3) Purification of GST-human procathepsin L protein

The E. coli Y1091 strain containing the plasmid for the expression of GST-human procathepsin L fusion gene was grown in a LB culture medium until its growth attained the middle or latter stage of the logarithmic phase, after which IPTG was added to the medium to a content of 0.5 mM followed by 4 hour fermentation to yield a GST-human procathepsin L fusion protein.

Then, the culture solution of the E. coli Y1091 strain mentioned above was centrifuged to collect E. coli cells which were suspended into a buffer solution A (20 mM of Tris-HCl, pH 8.0, + 5 mM of EDTA), and then crushed with an ultrasonic crusher (Sonifier 250 manufactured by Branson Co., Ltd.). The resulting solution of the sonicated cells was centrifuged at 1,000 ×g to provide the GST-human procathepsin L fusion protein as the sediment fraction.

This sediment fraction was suspended into another buffer solution A, after which a saturated solution of urea was added to the solution to a final concentration of 8 M. Then, 2-mercaptoethanol was added to the solution to a content of 1% (v/v), and then the mixture was allowed to stand at 60°C for 1-4 hours to solubilize the sediment.

The solution after solubilization was passed through an anion exchange resin (DEAE TOYO PEARL 650S manufactured by Toso Co., Ltd.) equilibrated with a buffer solution B (20 mM of Tris-HCl, pH 8.0, + 6M of urea), and the adsorbed constituents were eluted with a 0-0.3M gradient concentration of sodium chloride. The GST-human procathepsin L fusion protein-containing fractions were collected out of the resulting eluates, and concentrated by ultrafiltration (using Centriprep-30 manufactured by Amicon Co., Ltd.). Then the concentrated solution was subjected to gel filtration using TSK gel (G3000SW manufactured by Toso CO., Ltd.) equilibrated with a buffer solution C (50 mM of Na-Pi, pH 6.4, + 6M of urea + 0.5M of NaCl + 2% DMSO). Out of the resulting eluates, only the fractions which contained the GST-human procathepsin L fusion protein were collected and subjected to ultrafiltration by which a buffer solution A was substituted for the solvent to provide an antigenic protein (Preparation 1; hereunder this will be abbreviated as GST-L in some cases.) In the same manner(i.e. the above gene manipulations and protein purification), the fused protein of GST and the pro segment of human procathepsin L (Preparation 2; hereunder this will be abbreviated as GST-Pro L on occasion.) was also purified.

Preparations 3 - 7

Preparation of synthetic peptides of human procathepsin L

On the basis of the base sequence of human procathepsin L (Biochem. J., Vol.253, pp.303-306,1988)-(see the List OF SEQUENCES given later), the following peptide were synthesized out of the primary amino acid sequence of human procathepsin L:

a peptide consisting of the 94th to 107th amino acid sequence: Gly-Phe-Gln-Asn-Arg-Lys-Pro-Arg-Lys-Gly-Lys-Val-Phe-Gln (Preparation 3);

a peptide consisting of the 112th to 125th amino acid sequence: Tyr-Glu-Ala-Pro-Arg-Ser-Val-Asp-Trp-Arg-Glu-Lys-Gly-Tyr (Prepation 4);

a peptide consisting of the 282nd to 295th amino acid sequence: Gly-Tyr-Gly-Phe-Gln-Ser-Thr-Glu-Ser-Asp-Asn-Asn-Lys-Tyr (Preparation 5);

a peptide consisting of the 297th to 310th amino acid sequence: Leu-Val-Lys-Asn-Ser-Trp-Gly-Glu-Glu-Trp-Gly-Met-Gly-Gly (Preparation 6); and

a peptide consisting of the 308th to 321st amino acid sequence: Met-Gly-Gly-Tyr-Val-Lys-Met-Ala-Lys-Asp-Arg-Arg-Asn-His (Preparation 7), all the positions being in the LIST Of SEQUENCES given later.

Further, part of each of the respective resulting peptide was combined with a carrier protein BSA by the MBS method and used for immunological purposes.

Human procathepsin L is composed of pro and mature segments. Of the amino acid sequence of human procathepsin L, the amino acid sequence of Preparation 3 is involved in the pro segment of human procathepsin L, that of Preparation 4 harbors the boundary segment between the pro and mature segments, whereas those of Preparations 5 - 7 are involved in the mature segment of human procathepsin L.

Example 1

1) Preparation of hybridomas

(1) Immunity of mice

The GST-human procathepsin L protein from the above Preparation 1 was mixed with an equiamount of Freund's complete adjuvant followed by emulsification process. The resulting emulsion was administered into the abdomen of mice (BALB/c, 8 week old female). Two weeks later the GST-human procathepsin L protein was mixed with Freund's incomplete adjuvant at an equiamount rate to prepare an emulsion which was then used to give additional immunity to the mice. Only each antigen was administered to the mice via caudal vein three or four days prior to the start of the cell fusion mentioned later .

(2) Cell fusion

A spleen was removed from each mouse immunized as mentioned above, three or four days later after the above last immunity and then crushed with a homogenizer, after which the spleen cells were suspended in PBS. Next, the resulting suspension was adjusted so that it contained the spleen cells and myeloma cells (SP2/O-Ag14) at a ratio of 5:1, and then allowed to stand in the presence of 50% of polyethylene glycol for 3 minutes. Thereafter the suspension was centrifuged at 1,200 rpm for 8 minutes to remove off the supernatant, and gradual addition of 25 m1 of RPMI 1640 culture medium was made to the residue which was further centrifuged at 1,200 rpm for 8 minutes.

Next, the HAT RPMI 1640 culture medium with 10% FCS added was adjusted to make the final content of spleen cells $3.5 \times 10^6$/m1, and then poured in a 96 well microplate at a rate of 0.1 m1/well. This 96 well microplate was cultured in the presence of 5% $CO_2$ at 37 °C.

Two or three days after the start of culturing there was added to each well 0.1 m1 of HAT RPMI 1640 culture medium which contained 10% FCS, and half of the culture media were made fresh every 3 or 4 days. The formation of colonies was observed 7 to 10 days after the start of culturing, and in at least one well an antibody was produced which was sufficiently responsive to the immunogen. With the culture supernatant from this well in which the antibody was produced, the screening for the antibodies was made according to the following procedures:

(3) Screening

This screening for the antibodies was effected by the enzyme antibody technique (ELISA method; Immunochemistry, Vol.8, pp. 871-874, 1971).

Fifty $\mu$l of the culture supernatant was poured in a 96 well microplate having beforehand adsorbed thereon 50 $\mu$l of an antigen solution which was prepared through suspension with PBS. The mixture was then subjected to a 2 hour reaction at 30°C, and further to a 1 hour reaction at 30°C with a peroxidase-labeled anti-mouse antibody. Last, the selection of wells was made depending on the coloring observed when O-phenylenediamine was used as the substrate.

(4) Cloning

The cells which were harvested from the wells which were determined to be positive as a result of the screening, were subjected to cloning by the limiting dilution method. That is, 0.2 m1 of HT RPMI 1640 culture medium to which 10% FCS was added and which contained suspended cells composed of a hybridoma (5 cells/m1) and a thymocyte feeder ($1.0 \times 10^6$ cells/m1)was poured into each well of a 96 well microplate, and the culturing was done at 37°C for about 7-10 days. Only antibody-positive cells in the wells in which only one colony was formed were determined to be the object monoclonal antibody-producing hybridoma.

Thus, three strains of the object monoclonal antibody-producing hybridoma for human procathepsin L (RL-1, RL-2 and RL-3) were obtained. One strain of the object monoclonal antibody-producing hybridoma for the synthetic peptide of Preparation 3 (SLP1-2), one strain for the synthetic peptide of Preparation 4 (SLM3-2), two strains for the synthetic peptide of Preparation 5 (SLM1-2 and SLM1-3), one strain for the synthetic peptide of Preparation 6 (SLM4-1) and one strain for the synthetic peptide of Preparation 7 (SLM2-1) were harvested in the same manner as for the GST-human procathepsin L protein, except that the synthetic peptidase of Preparations 3-7 were each used instead of said protein.

2) Production and purification of monoclonal antibodies

Each of the above hybridomas was well grown in the 96 well microplate, and then transferred successively to 24 and 6 well microplates and a flask to gradually upscale the culturing. Each monoclonal antibody was thereafter purified from the culture supernatant using a protein G-Sepharose affinity column (manufactured by Pharmacia Co., Ltd.).

The identification of the classes and subclasses of the immunoglobulins corresponding to the monoclonal antibodies was made using a monoclonal antibody-subtyping kit (manufactured by BIORAD Co., Ltd.) The results are shown in Table 1.

TABLE 1

| Immunogen | Hybridoma strain | Subclass |
|---|---|---|
| GST-L (Preparation 1) | RL-1 | IgG2a |
|  | RL-2 | IgG1 |
|  | RL-3 | IgG2a |
| Synthetic peptides |  |  |
| (Preparation 3) | SLP1-2 | IgG2a |
| (Preparation 4) | SLM3-2 | IgG2b |
| (Prataration 5) | SLM1-2 | IgG2a |
|  | SLM1-3 (FERM BP-3998) | IgG2b |
| (Preparation 6) | SLM4-1 (FERM BP-3999) | IgG2a |
| (Preparation 7) | SLM2-1 | IgG1 |

3) Specificity of the monoclonal antibodies

(1) ELISA method (enzyme antibody technique)

Each of the nine types of monoclonal antibodies obtained as mentioned above was examined by the ELISA method in its reactivity to human procathepsin L protein and the synthetic peptides. That is, 50 $\mu$l of the culture supernatant of a sample of each antibody-producing hybridoma strain was transferred to a 96 well microplate with an antigen (GST-L, GST-ProL, GST or the synthetic protein of each of the Preparations 3 - 7) adsorbed beforehand thereon, and the mixture was then subjected to a 2 hour reaction at 30°C, and further to an additional hour of reaction at 30°C with a peroxidase-labeled anti-mouse antibody. Next, the reaction was further conducted in the presence of O-phenylenediamine substrate for 10 minutes.

Thus, as Table 2 shows, it has been confirmed that RL-1 from the GST-L antigen is reactive with the mature segment of the recombinant human procathepsin L, whereas RL-2 and RL-3 are reactive with the pro segment of the recombinant human procathepsin L. This means that RL-1, RL-2 and RL-3 are all reactive with human procathepsin L produced by recombinant E. coli. This fact was also evidenced by the Western blotting mentioned below. SLM3-2, SLM1-2, SLM1-3, SLM4-1 and SLM2-1 were confirmed to be reactive with the mature segment of the recombinant human procathepsin L.

In addition, the monoclonal antibodies purified from the respective culture supernatants were confirmed to have the same reaction properties as the above.

TABLE 2

| Culture supernatant | Antigein body | | | | | | | |
| | GST-L | GST-ProL | GST | Synthetic peptides | | | | |
| | Prep. 1 | Prep. 2 | | Prep. 3 | Prep. 4 | Prep. 5 | Prep. 6 | Prep. 7 |
|---|---|---|---|---|---|---|---|---|
| RL-1 | ++ | − | − | − | − | − | − | − |
| RL-2 | + | + | − | − | − | − | − | − |
| RL-3 | ++ | ++ | − | − | − | − | − | − |
| SLP1-2 | + | + | − | + | − | − | − | − |
| SLM3-2 | ++ | − | − | − | + | − | − | − |
| SLM1-2 | ++ | − | − | − | − | + | − | − |
| SLM1-3 | ++ | − | − | − | − | + | − | − |
| SLM4-1 | ++ | − | − | − | − | − | + | − |
| SLM2-1 | ++ | − | − | − | − | − | − | + |

(2) Western blotting

E. coli JM109 strain, which was prepared in Preparations 1 and 2 and produces only human procathepsin L, was treated at 100 °C for 2 minutes using a buffer for electrophoresis sampees, after which the electrophoresis was effected. The samples after electrophoresis were transferred to a membrane for Western blotting, and it was confirmed that the purified monoclonal antibodies RL-1, RL-2 and RL-3 are specifically reactive with human procathepsin L. Here, as the negative control, a version of JM109 strain was used which is free from the recombinant gene of cDNA for human procathepsin L.

It was established by Western blotting that the purified monoclonal antibodies RL-1, SLM3-2, SLM1-2, SLM1-3 and SLM4-1 obtained in Preparations 1, 4, 5 and 6, respectively, each had a specific reactivity to human kidney-derived cathepsin L (manufactured by Nova Biochem Co., Ltd.).

None of the nine types of monoclonal antibodies enumerated in Table 2 was reactive with either human liver cathepsin B (manufactured by Nova Biochem Co., Ltd.) or human leucocyte cathepsin G (manufactured by Nova Biochem Co., Ltd.) in Western blotting.

TABLE 3

| | RL-1 | RL-2 | RL-3 | SLP1-2 | SLM3-2 | SLM1-2 | SLM1-3 | SLM4-1 | SLM2-1 |
|---|---|---|---|---|---|---|---|---|---|
| Human kidney cathepsin L | ++ | − | − | − | + | + | + | + | − |
| Human liver cathepsin B | − | − | − | − | − | − | − | − | − |
| Human liver cathepsin G | − | − | − | − | − | − | − | − | − |

Example 2

Cathepsin L may be detected by utilizing the monoclonal antibodies to human procathepsin L according to the present invention.

As hereunder described, cathepsin L in the sera from healthy persons and that from cervical cancer patients were compared with each other and investigated by Western blotting procedures.

The sera from healthy persons and cervical cancer patients were treated at 100°C for 2 minutes using a buffer for electrophoresis samples, after which the electrophoresis was effected. The samples after electrophoresis was transferred to a membrane for Western blotting, and was then reacted with the purified monoclonal antibody SLM1-3 followed by reaction with alkaline phosphatase-labeled rabbit anti-mouse IgG antibody (Zymed Co., Ltd.), and then the coloring was done.

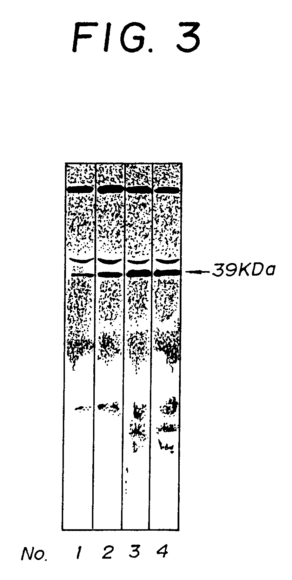

Thus, as is shown in Fig.3, the specimens from both the healthy persons (No. 1 and No.2) and the cervical cancer patients (No.3 and No.4) reacted with the monoclonal antibody at the band of 39 KD corresponding to human procathepsin L, and the reactivity was more intensive in the case of the cervical cancer patients.

Example 3

Detection of cathepsin L in the sera from cancer patients and healthy persons

The quantity of cathepsin L in the sera from various cancer-carrying patients and healthy persons was determined in order to investigate its usefulness as a cancer marker.

1) Preparation of assay plate

SLM1-3 antibody was diluted with PBS (phosphate-bufferred physiological saline) to a concentration of 1 $\mu$l/m1; the concentration may range from 0.1 to 5 $\mu$l/m1. The solution was poured in each well of a 96 well microplate (manufactured by Coning Co., Ltd.) and allowed to stand at 30°C for 2 hours or at 4°C overnight to bind the antibody to the plate. Washing was done with PBS containing 0.05% Tween (PBS-Tween), after which 320 $\mu$l of a Block Ace solution (Dai-nippon Pharmaceuticals Co. ,Ltd.) was poured into each well, and then the mixture was allowed to stand for blocking.

2) Detection of human serum cathepsin L

The sera from various cancer-carrying patients and healthy persons were diluted 50-fold with PBS; the dilution degree may be within the range of 10 to 100-fold. Fifty ml of each diluted serum was transferred to each well of the assay plate prepared in 1) above, and the reaction was allowed to proceed at 30°C for 2 hours. Washing was done with PBS-Tween, and then 50 $\mu$l of the peroxidase-labeled purified monoclonal antibody SLM4-1 (diluted to a concentration of 1-5$\mu$g/m1 with PBS) was added to the assay plate followed by an additional hour of reaction. The assay plate was then washed with PBS-Tween, after which 100$\mu$l of a peroxidase substrate solution was added to the plate for coloring. The substrate solution was prepared by dissolving 10 mg of O-phenylenediamine in 25 m1 of a 0.1M citrate-phosphate buffer solution (pH 5.0) followed by addition of 5$\mu$l of a 30% $H_2O_2$ solution. Twenty (20)$\mu$l of 2M $H_2SO_4$ was added to the solution to stop the reaction 20 minutes later; this interval may range from 10 to 30 minutes. Absorbance was determined at 492 nm.

In this way the determination was made of sera from healthy persons (8 specimens), those from cervical cancer patients (11 specimens) and those from ovarian cancer patients (45 specimens). The results are shown in Table 4.

TABLE 4

| Group | Number of specimens | Positive rate* | Early cancer positive rate** |
|---|---|---|---|
| Healthy women | 8 | | |
| Overian fumour patients | 45 | 40/45 (89%) | 3/4 (75%) |
| Cervical cancer patients | 11 | 7/11 (64%) | 2/5 (40%) |

* "Positive" assigned to the specimens which showed values over "the average of the healthy persons + 2 x standard deviation".
** Positive rate of early cancers (stage I).

As is shown in Table 4, both the sera from ovarian fumour patients and those from cervical cancer patients showed significantly higher values than those from healthy persons; the positive rates were as high as 89% in the ovarian fumour patients and 64% in the cervical cancer patients in the case where those specimens showing absorbance values over "the average of the healthy persons + 2 x standard deviation" were determined to be positive. Further, the positive rates were 75% in the early ovarian cancer (stage I) patients and 40% in the early cervical cancer (stage I) patients.

For the foregoing, the systems for detecting human cathepsin L including human procathepsin L according to the present invention are useful to diagnose cancers which are supposed to be related with cathepsin L, such as, for example, ovarian cancer, cervical cancer, etc., and further they are applicable also to the early diagnosis of cancers.

In conclusion, it is possible to specifically detect human cathepsin L including human procathepsin L by utilizing the monoclonal antibodies according to the present invention. Accordingly, those antibodies may be utilized for the diagnosis and therapy of diseases related with human cathepsin L including human procathepsin L.

LIST OF SEQUENCES

SEQUENCE ID NO.: 1

SEQUENCE LENGTH: 1573

SEQUENCE TYPE:    nucleic acid

STRANDEDNESS:    double

TOPOLOGY:        linear

MOLECULAR TYPE:  cDNA to mRNA

ORIGINAL SOURCE

      ORGANISM: Homo sapiens (human)

      TISSUE SOURCE: fibroblast

      LIBRARY:        GM637

FEATURE

      FEATURE KEY:    mRNA

      LOCATION:        1..1573

      FEATURE KEY:    CDS

      LOCATION:        289..1287

SEQUENCE

```
AGAACCGCGA CCTCCGCAAC CTTGAGCGGC ATCCGTGGAG TGCGCCTGCA GCTACGACCG    60

CAGCAGGAAA GCGCCGCCGG CCAGGCCCAG CTGTGGCCGG ACAGGCACTG GAAGAGAGGA   120

CGCGGTCGAG TAGGTGTGCA CCAGCCCTGG CAACGAGAGC GTCTACCCCG AACTCTGCTG   180

GCCTTGAGGT GGGGAAGCCG GGGAGGGCAG TTGAGGACCC CGCGGAGGCG CGTGACTGGT   240

TGAGCGGGCA GGCCAGCCTC CGAGCCGGGT GGACACAGGT TTTAAAAC ATG AAT CCT   297
                                                     Met Asn Pro
                                                      1

ACA CTC ATC CTT GCT GCC TTT TGC CTG GGA ATT GCC TCA GCT ACT CTA   345
Thr Leu Ile Leu Ala Ala Phe Cys Leu Gly Ile Ala Ser Ala Thr Leu
         5                  10                  15
```

```
ACA TTT GAT CAC AGT TTA GAG GCA CAG TGG ACC AAG TGG AAG GCG ATG    393
Thr Phe Asp His Ser Leu Glu Ala Gln Trp Thr Lys Trp Lys Ala Met
 20                  25                  30                  35

CAC AAC AGA TTA TAC GGC ATG AAT GAA GAA GGA TGG AGG AGA GCA GTG    441
His Asn Arg Leu Tyr Gly Met Asn Glu Glu Gly Trp Arg Arg Ala Val
                    40                  45                  50

TGG GAG AAG AAC ATG AAG ATG ATT GAA CTG CAC AAT CAG GAA TAC AGG    489
Trp Glu Lys Asn Met Lys Met Ile Glu Leu His Asn Gln Glu Tyr Arg
                 55                  60                  65

GAA GGG AAA CAC AGC TTC ACA ATG GCC ATG AAC GCC TTT GGA GAC ATG    537
Glu Gly Lys His Ser Phe Thr Met Ala Met Asn Ala Phe Gly Asp Met
                 70                  75                  80

ACC AGT GAA GAA TTC AGG CAG GTG ATG AAT GGC TTT CAA AAC CGT AAG    585
Thr Ser Glu Glu Phe Arg Gln Val Met Asn Gly Phe Gln Asn Arg Lys
     85                  90                  95

CCC AGG AAG GGG AAA GTG TTC CAG GAA CCT CTG TTT TAT GAG GCC CCC    633
Pro Arg Lys Gly Lys Val Phe Gln Glu Pro Leu Phe Tyr Glu Ala Pro
100                 105                 110                 115

AGA TCT GTG GAT TGG AGA GAG AAA GGC TAC GTG ACT CCT GTG AAG AAT    681
Arg Ser Val Asp Trp Arg Glu Lys Gly Tyr Val Thr Pro Val Lys Asn
                    120                 125                 130

CAG GGT CAG TGT GGT TCT TGT TGG GCT TTT AGT GCT ACT GGT GCT CTT    729
Gln Gly Gln Cys Gly Ser Cys Trp Ala Phe Ser Ala Thr Gly Ala Leu
                    135                 140                 145

GAA GGA CAG ATG TTC CGG AAA ACT GGG AGG CTT ATC TCA CTG AGT GAG    777
Glu Gly Gln Met Phe Arg Lys Thr Gly Arg Leu Ile Ser Leu Ser Glu
                    150                 155                 160
```

```
CAG AAT CTG GTA GAC TGC TCT GGG CCT CAA GGC AAT GAA GGC TGC AAT        825
Gln Asn Leu Val Asp Cys Ser Gly Pro Gln Gly Asn Glu Gly Cys Asn
    165             170             175

GCT GGC CTA ATG GAT TAT GCT TTC CAG TAT GTT CAG GAT AAT GGA GGC        873
Gly Gly Leu Met Asp Tyr Ala Phe Gln Tyr Val Gln Asp Asn Gly Gly
180             185             190             195

CTG GAC TCT GAG GAA TCC TAT CCA TAT GAG GCA ACA GAA GAA TCC TGT        921
Leu Asp Ser Glu Glu Ser Tyr Pro Tyr Glu Ala Thr Glu Glu Ser Cys
                200             205             210

AAG TAC AAT CCC AAG TAT TCT GTT GCT AAT GAC ACC GGC TTT GTG GAC        969
Lys Tyr Asn Pro Lys Tyr Ser Val Ala Asn Asp Thr Gly Phe Val Asp
                215             220             225

ATC CCT AAG CAG GAG AAG GCC CTG ATG AAG GCA GTT GCA ACT GTG GGG        1017
Ile Pro Lys Gln Glu Lys Ala Leu Met Lys Ala Val Ala Thr Val Gly
                230             235             240

CCC ATT TCT GTT GCT ATT GAT GCA GGT CAT GAG TCC TTC CTG TTC TAT        1065
Pro Ile Ser Val Ala Ile Asp Ala Gly His Glu Ser Phe Leu Phe Tyr
    245             250             255

AAA GAA GGC ATT TAT TTT GAG CCA GAC TGT AGC AGT GAA GAC ATG GAT        1113
Lys Glu Gly Ile Tyr Phe Glu Pro Asp Cys Ser Ser Glu Asp Met Asp
260             265             270             275

CAT GGT GTG CTG GTG GTT GGC TAC GGA TTT GAA AGC ACA GAA TCA GAT        1161
His Gly Val Leu Val Val Gly Tyr Gly Phe Glu Ser Thr Glu Ser Asp
                280             285             290

AAC AAT AAA TAT TGG CTG GTG AAG AAC AGC TGG GGT GAA GAA TGG GGC        1209
Asn Asn Lys Tyr Trp Leu Val Lys Asn Ser Trp Gly Glu Glu Trp Gly
                295             300             305
```

```
ATG GGT GGC TAC GTA AAG ATG GCC AAA GAC CGG AGA AAC CAT TGT GGA     1257

Met Gly Gly Tyr Val Lys Met Ala Lys Asp Arg Arg Asn His Cys Gly

      310                 315                 320

ATT GCC TCA GCA GCC AGC TAC CCC ACT GTG TGA GCTGGTGGAC GGTGATGAGG  1310

Ile Ala Ser Ala Ala Ser Tyr Pro Thr Val

    325                 330

AAGGACTTGA CTGGGGATGG CGCATGCATG GGAGGAATTC ATCTTCAGTC TACCAGCCCC  1370

CGCTGTGTCG GATACACACT CGAATCATTG AAGATCCGAG TGTGATTTGA ATTCTGTGAT  1430

ATTTTCACAC TGGTAAATGT TACCTCTATT TTAATTACTG CTATAAATAG GTTTATATTA  1490

TTGATTCACT TACTGACTTT GCATTTTCGT TTTTAAAAGG ATGTATAAAT TTTTACCTGT  1550

TTAAATAAAA TTTAATTTCA AATGT                                        1575
```

## Claims

1. A monoclonal antibody which is reactive specifically with human procathepsin L or with any of the following peptides corresponding to parts of the primary amino acid sequence of human procathepsin L:

a peptide comprising the 94th to 107th amino acid sequence: Gly-Phe-Gln-Asn-Arg-Lys-Pro-Arg-Lys-Gly-Lys-Val-Phe-Gln;

a peptide comprising the 112th to 125th amino acid sequence: Tyr-Glu-Ala-Pro-Arg-Ser-Val-Asp-Trp-Arg-Glu-Lys-Gly-Tyr;

a peptide comprising the 282nd to 295th amino acid sequence: Gly-Tyr-Gly-Phe-Glu-Ser-Thr-Glu-Ser-Asp-Asn-Asn-Lys-Tyr;

a peptide comprising the 297th to 310th amino acid sequence: Leu-Val-Lys-Asn-Ser-Trp-Gly-Glu-Trp-Gly-Met-Gly-Gly; and

a peptide comprising the 308th to 321st amino acid sequence: Met-Gly-Gly-Tyr-Val-Lys-Met-Ala-Lys-Asp-Arg-Arg-Asn-His.

2. A hybridoma characterized by producing the monoclonal antibody according to Claim 1.

3. A method of detecting human cathepsin L characterized by employing the monoclonal antibody according to Claim 1 to detect human cathepsin L in a specimen from a human.

4. The method of detecting human cathepsin L according to Claim 3, wherein the human is a patient suffering from a cancer connected with human cathepsin L, such as bronchogenic cancer, pancreatic cancer, hepatic cancer, cervical cancer, ovarian tumour or the like, or from a disease connected with aging such as osteoporosis.

# FIG. 1

# FIG. 2

# FIG. 3

←39KDa

No. 1 2 3 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | Section Ch, Week 8842,<br>Derwent Publications Ltd., London, GB;<br>Class B04, AN 88-300018<br>& US-A-7 154 692 (US DEPT. HEALTH & HUMAN)<br>6 September 1988<br>* abstract *<br>--- | 1-4 | C12P21/08<br>C12N5/20<br>G01N33/577<br>G01N33/68<br>//C12N15/57 |
| X | EMBL Database;<br>Accession number P82094; 26 October 1990<br>M. Gottesman et al.:"Cloned deoxyribo-<br>nucleic acid for human procathepsin L -<br>used for producing protein and antibodies<br>and for detection of tumour activity.<br>* abstract *<br>& US-A-7 154 692 (US DEPT. HEALTH & HUMAN)<br>--- | 1-4 | |
| A | NUCLEIC ACIDS RESEARCH<br>vol. 15, no. 7, 1987, LONDON, GB<br>page 3186<br>L. JOSEPH ET AL. 'The major ras induced<br>protein in NIH3T3 cells is cathepsin L.'<br>* the whole document * | 1-4 | |

TECHNICAL FIELDS
SEARCHED (Int. Cl.5 )

C12P
C12N
G01N
C07K

The present search report has been drawn up for all claims

| Place of search | Date of completion of the search | Examiner |
|---|---|---|
| THE HAGUE | 10 JUNE 1993 | NOOIJ F.J.M. |

EPO FORM 1503 03.82 (P0401)